**Europäisches Patentamt**

**European Patent Office**

**Office européen des brevets**

(19)

(11) Veröffentlichungsnummer: **0 022 888**
A1

(12)

# EUROPÄISCHE PATENTANMELDUNG

(21) Anmeldenummer: 79810089.7

(22) Anmeldetag: 12.09.79

(51) Int. Cl.³: **G 03 B 41/16**
A 61 B 6/14

(30) Priorität: 24.07.79 CH 6869 79

(43) Veröffentlichungstag der Anmeldung:
28.01.81 Patentblatt 81/4

(84) Benannte Vertragsstaaten:
BE DE FR GB IT LU NL SE

(71) Anmelder: Hawe-Neos Dental Dr. H. v. Weissenfluh AG

CH-6925 Gentilino(CH)

(72) Erfinder: von Weissenfluh, Hans, Dr.

CH-6925 Gentilino(CH)

(74) Vertreter: Baggiolini, Raimondo
Racheli & Fiammenghi Via San Gottardo 15
CH-6900 Lugano(CH)

(54) **Hilfsvorrichtung für Röntgenaufnahmen von Zähnen.**

(57) Um bei Röntgenaufnahmen von Zähnen die Achse der Röntgenröhre senkrecht zur Winkelhalbierenden zwischen der Zahnachse und dem Filmträger einstellen zu können, ist ein Gelenkrahmen mit einem Stab und einem Zeiger mit dem Filmträger verbunden. Der Zeiger wird auf die Zahnachse eingestellt, wobei in der einfachsten Ausführungsform der Winkel zwischen dem Zeiger und dem Filmträger anhand einer Skala abgelesen und dann der Zeiger auf den halben Winkelwert eingestellt wird, so dass der Stab die Richtung anzeigt, in der die Röntgenröhrenachse liegen soll. In einer bevorzugten Ausführungsform ist ein Gelenkviereck G-H-I-K vorgesehen, bei dem die eine Seite G-H doppelt so lang wie die gegenüberliegende Seite I-K ist. Bei dieser Ausführungsform weist der Stab 4 automatisch in die auf die Winkelhalbierende senkrecht stehende Richtung, wenn der Zeiger 9 auf die Zahnachse eingestellt wird. Die Achse eines Ringes 8, der mit dem Stab 4 starr verbunden ist, kann vorgesehen sein, um die durch den Filmträger 1 verlaufende Röntgenröhrenachse E-F anzugeben.

Fig 4

EP 0 022 888 A1

- 1 -

Hilfsvorrichtung für Röntgenaufnahmen von Zähnen

Die Erfindung betrifft eine Hilfsvorrichtung für Röntgenaufnahmen von Zähnen.

Beim Röntgen eines Zahnes legt der Zahnarzt einen Filmträger hinten an den zu röntgenden Zahn an und richtet
die Röntgenröhre so aus, dass eine möglichst verzerrungsfreie Aufnahme erreicht wird. Diese Richtung der Röntgenröhre lässt sich aber nur schwer bestimmen, da der Film
niemals parallel zur Zahnachse gelegt werden kann. Dies
ist durch den Umstand bedingt, dass der Filmträger bei
der Aufnahme eines Zahnes aus dem Oberkiefer beispielsweise mit der unteren Seite auf dem zu röntgenden Zahn
aufliegen und mit der oberen Seite am Gaumen anliegen
muss, wenn er so gross sein soll, dass auch die Zahnwurzel geröntgt werden kann.

Es lässt sich nun theoretisch nachweisen, und die Praxis hat dies bestätigt, dass eine verzerrungsarme Rönt-

- 2 -

genaufnahme dann erreicht wird, wenn die Achse der
Röntgenröhre senkrecht zur Winkelhalbierenden desjenigen Winkels verläuft, der von der Zahnachse und
der Filmebene eingeschlossen wird.

Der Erfindung liegt nun die Aufgabe zugrunde, eine
Hilfsvorrichtung zu schaffen, mit der die Röntgenröhrenachse senkrecht zu der Winkelhalbierenden zwischen der Zahnachse und der Filmebene eingestellt
werden kann.

Diese Aufgabe wird erfindungsgemäss dadurch gelöst,
dass ein Gelenkarm mit einem Filmträger verbunden
ist und einen Stab aufweist, der die Richtung der
Röntgenröhrenachse in Abhängigkeit von dem Winkel angibt, der durch die Achse des zu röntgenden Zahnes
und die Ebene des Filmträgers gebildet wird.

Eine erste Ausführungsform der Erfindung ist dadurch
gekennzeichnet, dass der Filmträger mit Hilfe eines
Verbindungsstückes fest mit einer Gradskala verbunden ist, dass ein auf die Zahnachse einstellbarer
Zeiger über die Gradskala schwenkbar am Verbindungsstück gelagert ist und dass der Stab im rechten Winkel starr mit dem Zeiger verbunden ist.

Bei dieser Ausführungsform wird zunächst der Winkel
zwischen der Zahnachse und der Filmebene ermittelt,
indem der Zeiger auf die Zahnachse eingestellt wird.
Danach rechnet man sich den halben Winkelwert aus und
stellt den Zeiger auf diesen Wert ein, so dass der
Stab nunmehr die Richtung anzeigt, in der die Röntgenröhrenachse verlaufen soll.

Ein zweites Ausführungsbeispiel der Erfindung ist dadurch gekennzeichnet, dass der Gelenkrahmen ein Gelenkviereck umfasst, dessen erste Seite von einem Abschnitt des Stabes, der die Richtung der Röntgenröhrenachse angibt, gebildet ist, und dessen zweite Seite, die parallel zu der ersten Seite verläuft und halb so lang wie die erste Seite ist, starr im rechten Winkel mit einem Zeiger verbunden ist, der auf die Zahnachse einstellbar ist, dass ein Verbindungsstück, das mit dem Filmträger fest verbunden ist, rechtwinkelig mit einem Gelenk an der zweiten Seite und am Zeiger befestigt ist und dass die Anordnung so getroffen ist, dass nach Anlegen des Filmträgers und nach dem Einstellen des Zeigers auf die Zahnachse der Stab od. dgl. die Richtung der Röntgenröhrenachse automatisch angibt, die senkrecht zu der Winkelhalbierenden des Winkels verläuft, der durch die Zahnachse und die Ebene des Filmträgers gebildet wird.

Bei diesem Ausführungsbeispiel wird, nachdem der Zeiger auf die Zahnachse eingestellt worden ist, automatisch die Richtung angegeben, in der die Röntgenachse verlaufen soll, ohne dass hierfür ein Winkel abgelesen und im Kopf halbiert werden müsste.

In den Unteransprüchen sind weitere vorteilhafte Ausgestaltungen der Erfindung angegeben.

- 4 -

Weitere Vorteile, Merkmale und Einzelheiten der Erfindung ergeben sich aus der nachstehenden, anhand der beiliegenden Zeichnung erfolgenden Beschreibung mehrerer Ausführungsbeispiele.

In der Zeichnung stellen dar:

Figur 1 schematisch die räumlichen Beziehungen zwischen einem Filmträger, einem Zahn und einer Röntgenröhre,

Figur 2 eine Seitenansicht eines ersten Ausführungsbeispiels der Erfindung,

Figur 3 eine Draufsicht auf die Ausführungsform der Figur 2,

Figur 4 eine perspektivische Ansicht einer zweiten Ausführungsform der Erfindung,

Figur 5 eine Seitenansicht des Ausführungsbeispieles der Figur 4 und

Figur 6 ein drittes Ausführungsbeispiel der Erfindung.

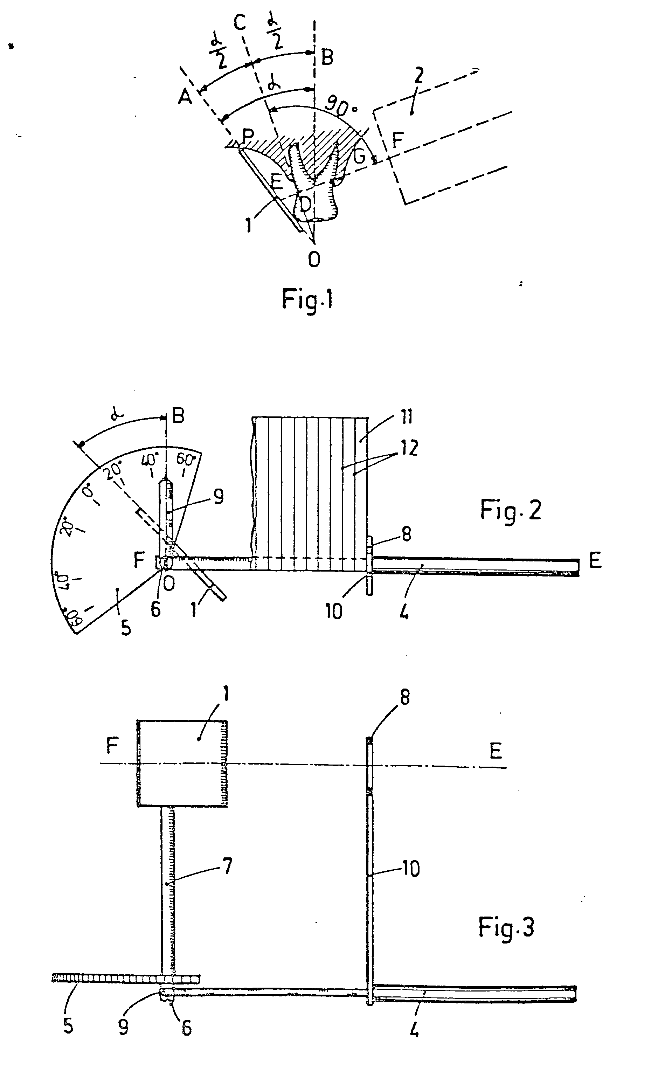

Es wird zunächst auf Figur 1 Bezug genommen. Mit 1 ist ein Filmträger bezeichnet, der theoretisch parallel zur Achse O-B des zu röntgenden Zahnes D liegen sollte, und mit 2 ist eine Röntgenröhre bezeichnet, deren Achse E-F senkrecht zur Achse O-B verlaufen sollte. In diesem Falle würde man ein verzerrungsfreies Zahnbild erhalten.

In der Praxis ist es jedoch nicht möglich, den Filmträger parallel zur Zahnachse O-B anzuordnen, da der Gau-

- 5 -

men P eine solche Lage des Filmträgers, der ja auch
eine gewisse Grösse haben muss, um die Zahnwurzel erfassen zu können, verhindert, wie dies aus Figur 1
hervorgeht.

Theorie und Praxis haben nun gezeigt, dass für eine
korrekte Röntgenaufnahme des Zahnes und der Wurzel
die Achse E-F der Röhre am besten senkrecht zur Winkelhalbierenden O-C des Winkels $\alpha$ , der von der
Ebene A-O des Filmträgers 1 und der Achse O-B des
Zahnes eingeschlossen wird, ausgerichtet wird.

Mit der erfindungsgemässen Vorrichtung kann dieses
Erfordernis erfüllt werden.

Wie in den Figuren 2 und 3 veranschaulicht, ist an
einem Ende einer Verbindungsstange 7 der Filmträger 1
befestigt und an dem anderen Ende eine Gradskala 5.
Ein Zeiger 9, der mit einem im rechten Winkel zu ihm
stehenden Stab 4 starr verbunden ist, ist mit der
Verbindungsstange 7, wie dargestellt, gelenkig verbunden, so dass die Zeigerachse mit der Achse der
Verbindungsstange 7 zusammenfällt und durch den
Scheitel der kreissektorförmigen Gradskala 5 geht.
Vom Stab 4 steht im rechten Winkel ein Arm 10 ab, an
dessen freiem Ende ein Ring 8 vorgesehen ist, dessen
Achse parallel zum Stab 4 verläuft.

Diese Ausführungsform der Erfindung wird wie folgt
gebraucht:

- 6 -

Der Filmträger 1 wird mit einem Film bestückt, an den zu röntgenden Zahn angelegt und der Zeiger 9 auf die Zahnachse O-B eingestellt. Auf der Gradskala 5 kann nun der Winkel $\alpha$, der vom Filmträger 1 und der Zahnachse O-B gebildet wird, gemessen werden. Der gemessene Winkel wird dann halbiert und der Zeiger 9 auf den halbierten Winkelwert eingestellt, so dass der Stab 4, der auf dem Zeiger 9 senkrecht steht, die korrekte Richtung der Röntgenröhrenachse E-F angibt. Die Achse des Ringes 8 trifft dabei auf den Filmträger 1 auf und stimmt mit der Röntgenröhrenachse E-F überein.

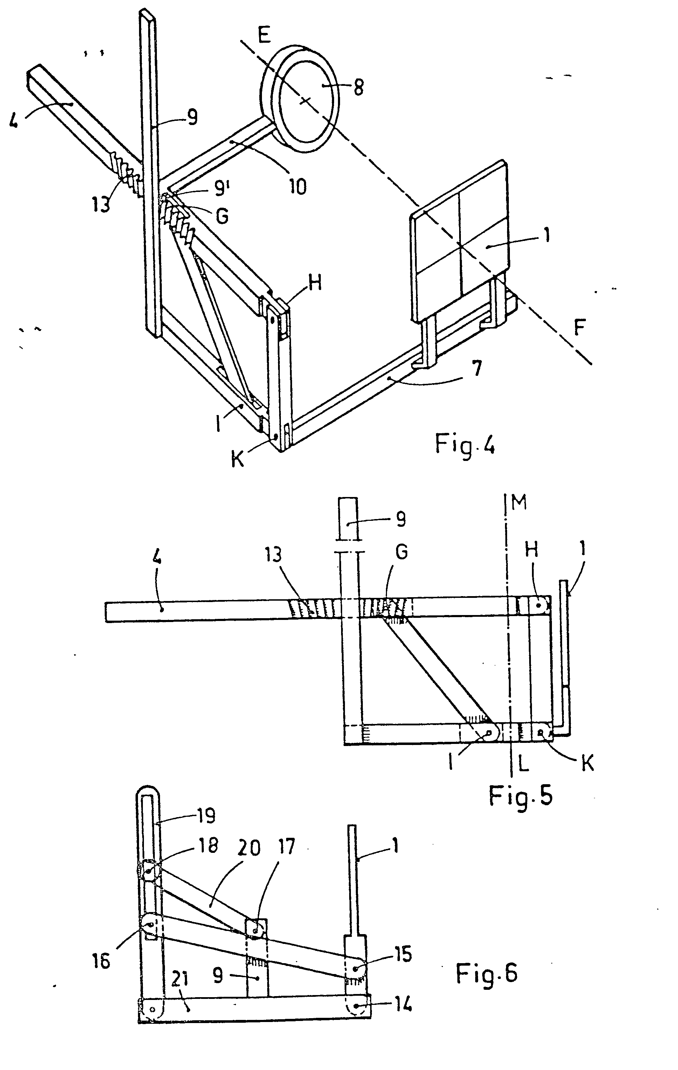

In den Figuren 4 und 5 ist eine Ausführungsform der Erfindung gezeigt, die zwar komplizierter als die vorangegangene Ausführungsform ist, jedoch ein Rechnen, nämlich das Halbieren des Winkels, überflüssig macht. Bei dieser Vorrichtung wird automatisch die korrekte Stellung der Röntgenröhrenachse angegeben, wie gross oder wie klein der Winkel $\alpha$ auch sein mag.

Bei der zweiten Ausführungsform ist, wie in den Figuren 4 und 5 gezeigt, ein ein Viereck bildender Gelenkrahmen vorgesehen, dessen erste Seite G-H einen Abschnitt des die korrekte Richtung E-F der Röntgenröhrenachse angebenden Stabes 4 bildet. Die zweite Seite I-K des Gelenkvierecks verläuft parallel zur ersten Seite G-H und ist halb so lang wie diese. Die zweite Seite I-K ist senkrechtstehend mit dem Zeiger 9 fest verbunden, der auf die Zahnachse O-B einstellbar ist. Die Verbindungsstange 7, an der der Filmträger 1 befestigt ist, steht senkrecht zum Zeiger 9 und der Seite I-K und ist über ein Gelenk in K befestigt. Der Stab 9 kann auch in der mit L-M in Figur 5 angegebenen Stellung angeordnet sein.

Aufgrund des Verhältnisses von 2 : 1 der Seiten G-H und I-K des Gelenkvierecks, das in Figur 5 gezeigt ist, wird der Winkel $\alpha$, der von der Ebene des Filmträgers und der Zahnachse eingeschlossen wird, durch zwei geteilt, wodurch der Stab 4 und damit der Ring 8, der über den Arm 10 starr mit dem Stab 4 verbunden ist, automatisch die zur Winkelhalbierenden des Winkels senkrecht stehende Richtung einnimmt, die mit der Richtung der Röntgenröhrenachse E-F übereinstimmt, ohne dass irgendein Rechenvorgang erforderlich wäre.

In einer modifizierten Ausführungsform, die in Figur 6 gezeigt ist, ist das Gelenkviereck G-H-I-K durch eine spezielle Anordnung von Armen ersetzt, wobei an den Punkten 14, 15, 16, 17, 18 Gelenke vorgesehen sind und das Gelenk 18 am Arm 20 in einem Schlitz 19 verschiebbar ist.

Der mit dem Arm 21 fest verbundene Stab 9 wird auf die Zahnachse eingestellt. Die Richtung der Röntgenröhrenachse wird dabei durch das Lot zum Schlitz 19 bestimmt, da der Schlitz 19 die Richtung der Winkelhalbierenden des Winkels $\alpha$ annimmt, der von dem Filmträger 1 und dem Stab 9 eingeschlossen wird.

Die einzelnen Teile der erfindungsgemässen Anordnung können unterschiedliche Formen aufweisen. Ferner kön-

- 8 -

nen verschiedene Materialien für die einzelnen Teile gewählt werden. Werden die Verbindungstange 7 mit dem Filmträger 1 und der Arm 10 mit dem Ring 8 in der Zeichnung links vom Stab 4 angeordnet, so dass die in Figur 4 spiegelbildliche Vorrichtung erhalten wird, so können ausser den vorderen Zähnen auch Zähne geröntgt werden, die auf der anderen Seite im Mund liegen.

In einer anderen, nicht dargestellten, modifizierten Ausführungsform ist der Ring 8 durch eine Zeigereinrichtung ersetzt, die die Richtung der Röntgenachse angibt.

0022888

- 1 -

Patentansprüche:

1. Hilfsvorrichtung für die Durchführung von Röntgenaufnahmen von Zähnen, dadurch gekennzeichnet, dass ein Gelenkrahmen (4, 7) mit einem Filmträger verbunden ist und einen Stab (4) aufweist, der die Richtung der Röntgenröhrenachse (E - F) in Abhängigkeit von dem Winkel angibt, der durch die Achse (O - B) des zu röntgenden Zahnes (D) und die Ebene des Filmträgers gebildet wird.

2. Vorrichtung nach Anspruch 1, dadurch gekennzeichnet, dass der Filmträger (1) mit Hilfe eines Verbindungsstückes (7) fest mit einer Gradskala (5) verbunden ist, dass ein auf die Zahnachse einstellbarer Zeiger (9) über die Gradskala (5) schwenkbar am Verbindungsstück (7) gelagert ist und dass der Stab (4) im rechten Winkel starr mit dem Zeiger (9) verbunden ist.

- 2 -

3. Vorrichtung nach Anspruch 1 oder 2, dadurch gekennzeichnet, dass der Stab (4) mit einem Arm (10) versehen ist, der einen Ring (8) oder eine andere Anzeigevorrichtung trägt, dessen Achse mit der Richtung der Röntgenröhrenachse übereinstimmt und durch den Mittelpunkt des Filmträgers (1) verläuft.

4. Vorrichtung nach Anspruch 1, dadurch gekennzeichnet, dass der Gelenkrahmen ein Gelenkviereck (G-H-I-K, Figuren 4 und 5) umfasst, dessen ersten Seite (G-H) von einem Abschnitt des Stabes (4), der die Richtung der Röntgenröhrenachse (E-F) angibt, gebildet ist, und dessen zweite Seite (I-K), die parallel zur ersten Seite verläuft und halb so lang wie die erste Seite (G-H) ist, starr im rechten Winkel mit einem Zeiger (9) verbunden ist, der auf die Zahnachse (O-B) einstellbar ist, dass ein Verbindungsstück (7), das mit dem Filmträger (1) fest verbunden ist, rechtwinkelig mit einem Gelenk (K) an der zweiten Seite (I-K) und am Zeiger (9) befestigt ist und dass die Anordnung so getroffen ist, dass nach Anlegen des Filmträgers (1) und nach dem Einstellen des Zeigers (9) auf die Zahnachse der Stab(4) od. dgl. die Richtung der Röntgenröhrenachse automatisch angibt, die senkrecht zur Winkelhalbierenden (O-C, Figur 1) des Winkels ($\alpha$) verläuft, der durch die Zahnachse und die Ebene (O-A) des Fimträgers gebildet wird.

0022888

- 3 -

5.  Vorrichtung nach Anspruch 4, dadurch gekennzeichnet, dass der Stab (4) starr mit einem Arm (10)
    verbunden ist, der einen Ring (8) trägt, dessen
    Achse mit der Richtung der Röntgenröhrenachse
    (E-F) übereinstimmt und durch den Mittelpunkt des
    Filmträgers (1) verläuft.

6.  Vorrichtung nach Anspruch 1, dadurch gekennzeichnet, dass Arme mit Hilfe von Gelenken (14, 15, 16,
    17, 18; Figur 6) derart miteinander verbunden sind,
    dass ein Viereck gebildet wird, wobei ein eine
    Seite des Vierecks bildender Arm (19) einen Schlitz
    aufweist, in dem ein Gelenkpunkt (18) verschiebbar
    ist und der derart verläuft, dass er die Richtung
    der Winkelhalbierenden des Winkels ($\alpha$) angibt, der
    von der Zahnachse (Stab 9) und der Ebene des Film-
    trägers (1) eingeschlossen wird.

0022888

Fig.1

Fig·2

Fig·3

Fig. 4

Fig. 5

Fig. 6

| Kategorie | Kennzeichnung des Dokuments mit Angabe, soweit erforderlich, der maßgeblichen Teile | betrifft Anspruch |
|---|---|---|
| | US - A - 2 127 502 (DE WAEL) <br> * Seite 1, rechte Spalte, Zeile 24 - Seite 3, linke Spalte, Zeile 58 * <br> -- | 1 |
| | US - A - 2 090 933 (BOLIN) <br> * Seite 1, linke Spalte, Zeile 47 - Seite 2, linke Spalte, Zeile 39 * <br> -- | 3-6 |
| | US - A - 2 123 210 (SCHANTZ) <br> * Seite 1, linke Spalte, Zeile 53 - Seite 2, linke Spalte, Zeile 19 * <br> -- | 2 |
| | DE - C - 719 672 (STEIN) <br> * Seite 2, Zeilen 17-93 * <br> -- | 1 |
| | DE - C - 642 156 (BRAUN) <br> * Seite 2, Zeilen 5-61 * <br> ---- | 1 |

**EINSCHLÄGIGE DOKUMENTE**

KLASSIFIKATION DER ANMELDUNG (Int Cl ⁺)

G 03 B 41/16
A 61 B 6/14

RECHERCHIERTE SACHGEBIETE (Int. Cl ⁺)

G 03 B 41/16
A 61 B 6/14

KATEGORIE DER GENANNTEN DOKUMENTE

X. von besonderer Bedeutung
A technologischer Hintergrund
O nichtschriftliche Offenbarung
P Zwischenliteratur
T der Erfindung zugrunde liegende Theorien oder Grundsatze
E kollidierende Anmeldung
D in der Anmeldung angefuhrtes Dokument
L. aus andern Grunden angefuhrtes Dokument
&: Mitglied der gleichen Patent-familie, übereinstimmendes Dokument

Der vorliegende Recherchenbericht wurde für alle Patentanspruche erstellt.

| Recherchenort, | Abschlußdatum der Recherche | Pruter |
|---|---|---|
| Den Haag | 21-10-1980 | MEES |

EPA form 1503.1 06.78

BAD ORIGINAL